# EUROPÄISCHE PATENTSCHRIFT

(11) **EP 2 285 779 B1**
(45) Veröffentlichungstag und Bekanntmachung des Hinweises auf die Patenterteilung: **27.03.2013**
(21) Anmeldenummer: 09742078.0
(22) Anmeldetag: 06.05.2009
(51) Int. Cl.: C07D 213/82, C07D 231/14, C07D 231/16, C07C 231/02, C07C 233/57, C07C 233/64

(54) **Verfahren zur Herstellung von Arylcarboxamiden**
Method for manufacturing aryl carboxamides
Procédé de production de carboxamides d aryle

(30) Priorität: 08.05.2008 EP 08155888
(43) Veröffentlichungstag der Anmeldung: 23.02.2011
(73) Patentinhaber: BASF SE, 67056 Ludwigshafen (DE)
(72) Erfinder: REICHERT, Wolfgang, 67227 Frankenthal (DE); KORADIN, Christopher, 67067 Ludwigshafen (DE); SMIDT, Sebastian Peer, 68723 Oftersheim (DE); MAYWALD, Volker, 67071 Ludwigshafen (DE); WOLF, Bernd, 67136 Fußgönheim (DE); RACK, Michael, 69214 Eppelheim (DE); ZIERKE, Thomas, 67459 Böhl-Iggelheim (DE); KEIL, Michael, 67251 Freinsheim (DE)
(86) Internationale Anmeldenummer: PCT/EP2009/055446
(87) Internationale Veröffentlichungsnummer: WO 2009/135860

(56) Entgegenhaltungen:
- EP-A- 0 657 438
- JP-A- 2001 172 276
- C. CATIVIELA, J. FERNANDEZ, E. MELENDEZ: "A Convenient Synthesis of N-Aryl-1,2-dihydro-2-oxo-3-pyridinecarboxa mides, N-Aryl-N-mehtyl-1,2-dihydro-2-oxo-3-pyridi necarboxamides and Their 1-Methyl (O-Methyl)-Derivatives." JOURNAL OF HETEROCYCLIC CHEMISTRY, Bd. 19, 1982, Seiten 1093-1097, XP002501342

## Beschreibung

Die vorliegende Erfindung betrifft ein Verfahren zur Herstellung von Arylcarboxamiden der Formel (I) 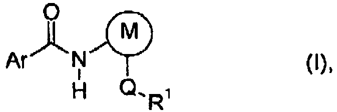 wobei die Substituenten folgende Bedeutungen haben:
- Ar: ein- bis dreifach substituierter Phenyl-, Pyridyl- oder Pyrazolylring, wobei die Substituenten unabhängig voneinander ausgewählt sind aus Halogen, C₁-C₄-Alkyl und C₁-C₄-Halogenalkyl;
- M: Thienyl oder Phenyl, das einen Halogensubstituenten tragen kann;
- Q: eine direkte Bindung, Cyclopropylen, ein annellierter Bicyclo[2.2.1]heptan- oder Bicyclo[2.2.1]hepten-Ring;
- R¹: Wasserstoff, Halogen, C₁-C₆-Alkyl, C₁-C₄-Alkoxy, C₁-C₄-Halogenalkoxy, ein- bis dreifach substituiertes Phenyl, wobei die Substituenten unabhängig voneinander ausgewählt sind aus Halogen und Trifluormethylthio, oder Cyclopropyl;
durch Umsetzung eines Säurechlorids der Formel (II) 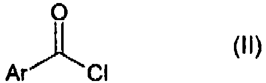 mit einem Arylamin (III) 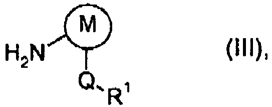 in einem geeigneten nicht-wässrigen Lösungsmittel, wobei man in Abwesenheit einer Hilfsbase
a) das Säurechlorid (II) vorlegt,
b) einen Druck von 0 bis 700 mbar einstellt,
c) das Arylamin (III) zudosiert, so dass das Molverhältnis von (II) zu (III) 0,9 : 1 bis 1,1 : 1 beträgt und
d) das Wertprodukt isoliert.

C. Cativiela et al. in Journal of Heterocyclic Chemistry, Bd. 19, 1982, S. 1093-1097, beschreibt ein Verfahren zur Herstellung von N-Phenyl-2-methoxy-3-pyridincaboxamid durch Umsetzung von 2-Chlornicotinsäurechlorid mit Anilin in 1,4-Dioxan. Die Umsetzung erfolgt bei Normaldruck, wobei das Säurechlorid zu der Aminlösung zugegeben wird.

Aus JP-A 2001/172276 ist bekannt, dass Alkyl- oder Phenylcarbonsäurechloride unter reduziertem Druck mit Arylaminen umgesetzt werden können. Die beschriebenen Reaktionen werden ohne Hilfsbase, jedoch in stark verdünnten Lösungen durchgeführt. Für eine großtechnische Herstellung der Arylcarboxamide (I) ist dieses Verfahren allerdings wegen der großen Lösungsmittelmengen ungeeignet. Eine konzentriertere Fahrweise ist nicht möglich, da diese zu Verklumpung und Durchmischungsproblemen führt, wodurch sich die Ausbeute an Wertprodukt stark verringert.

Andere in der Literatur beschriebene Verfahren zur Herstellung von Carboxamiden aus Säurechlorid und Arylamin ohne Verwendung einer Hilfsbase (vgl. z.B. Journal of Combinatorial Chemistry (2003), 5(3), 253-259 , Structural Chemistry (2006), 17(2), 241-247 und JP-A 1973/049217) sind großtechnisch nicht brauchbar, weil sie die gewünschten Wertprodukte nur in schlechten Ausbeuten liefern.

Der Erfindung lag demnach die Aufgabe zugrunde, ein großtechnisch brauchbares Verfahren zur Herstellung der Arylcarboxamide (I) bereitzustellen.

Demgemäß wurde gefunden, dass die Arylcarboxamide (I) in hohen Ausbeuten erhältlich sind, indem man in Abwesenheit einer Hilfsbase
a) das Säurechlorid (II) vorlegt,
b) einen Druck von 0 bis 700 mbar einstellt,
c) das Arylamin (III) in etwa stöchiometrischer Menge zudosiert und
d) das Wertprodukt isoliert.

Die Säurechloride (II) sind entweder kommerziell erhältlich oder können z.B. gemäß R. C. Larock, Comprehensive Organic Transformations, Verlage Wiley-VCH, 2nd Edition 1999, Seiten 1929 ff. hergestellt werden.

Die Arylamine (III) sind im allgemeinen durch Hydrierung der entsprechenden Nitroaromaten erhältlich. Nähere Angaben sind beispielsweise R. C. Larock, Comprehensive Organic Transformations, Verlage Wiley-VCH, 2nd Edition 1999, Seiten 821 ff. zu entnehmen.

Der Begriff "Halogen" bezeichnet jeweils Fluor, Chlor, Brom oder Jod, vorzugsweise Fluor oder Chlor;

"C₁-C₆-Alkyl", wie hierin verwendet, bezeichnet eine gesättigte, geradkettige oder verzweigte Kohlenwasserstoffgruppe, umfassend 1 bis 6 Kohlenstoffatome, speziell 1 bis 4 Kohlenstoffatome, beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isome re. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.

"C₁-C₄-Halogenalkyl" steht für einen teilweise oder vollständig halogenierten C₁-C₄-Alkyl-rest, wobei das/die Halogenatom(e) insbesondere Fluor, Chlor und/oder Brom ist/sind, also z.B. Chlormethyl, Brommethyl, Dichlormethyl, Trichlormethyl, Fluormethyl, Difluor-methyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2-Chlor-2-fluorethyl, 2,2,2-Trifluorethyl, 2-Chlor-1,1,2-trifluorethyl, 2-Chlor-2,2-difluorethyl, 2-Brom-2,2-difluorethyl, 2,2-Dichlor-2-fluorethyl, 2,2,2-Trichlorethyl, 1,1,2,2-Tetrafluorethyl, 1,1,2,2-Tetrachlorethyl, Pentafluorethyl, 2,2,3,3-Tetrafluor-1-propyl, 1,1,2,3,3,3-Hexafluor-1-propyl, 1,1,1,3,3,3-Hexafluor-2-propyl, Heptafluor-1-propyl, Heptafluor-2-propyl, 2,2,3,3,4,4,4-Heptafluor-1-butyl oder Nonafluor-1-butyl, insbesondere für Halogenmethyl, besonders bevorzugt für CH(F)₂ und CF₃;

"C₁-C₄-Alkoxy" steht für Methoxy, Ethoxy, n-Propoxy, 1-Methylethoxy, n-Butoxy, 1-Methylpropoxy, 2-Methylpropoxy oder 1,1-Dimethylethoxy, insbesondere für 1-Methylethoxy;

"C₁-C₄-Halogenalkoxy" steht für einen teilweise oder vollständig halogenierten C₁-C₄-Alkoxy-Rest, wobei das/die Halogenatom(e) insbesondere Fluor, Chlor und/oder Brom ist/sind, also z.B. OCH₂Cl, OCH₂Br, OCHCl₂, OC(Cl)₃, OCH₂F, OCHF₂, OCF₃, OCHFCl, OCFCl₂, OCF₂Cl, OCHCl-CH₃, OCHBr-CH₃, OCHF-CH₃, OCH₂-CH₂F, OCH₂-CHF₂, OCH₂-CHFCl, OCH₂-CF₃, OCF₂-CHFCl, OCH₂-CF₂Cl, OCH₂-CF₂Br, OCH₂-CFCl₂, OCH₂-C(Cl)₃, OCF₂-CHF₂, OC(Cl)₂-CHCl₂, OC₂F₅, OCH₂-CF₂-CHF₂, OCF₂-CHF-CF₃, OCH(CF₃)₂, O(n-C₃F₇), OCF(CF₃)₂,2,2,3,3,4,4,4-Heptafluor-1-butoxy oder Nonafluor-1-butoxy, insbesondere für OCF₂-CHF-CF₃.

Die Herstellung der folgenden Arylcarboxamide (I) ist bevorzugt:
Benodanil, Bixafen, Boscalid, Flutolanil, Mepronil, Penthiopyrad,
N-(2-Bicyclopropyl-2-yl-phenyl)-3-difluormethyl-1-methylpyrazol-4-yl-carboxamid,
N-(3',4',5'-Trifluorbiphenyl-2-yl)-1,3-dimethyl-pyrazol-4-yl-carboxamid,
N-(3',4',5'-Trifluorbiphenyl-2-yl)-1,3-dimethyl-5-fluor-pyrazol-4-yl-carboxamid,
N-(3',4',5'-Trifluorbiphenyl-2-yl)-5-chlor-1,3-dimethyl-pyrazol-4-yl-carboxamid,
N-(3',4',5'-Trifluorbiphenyl-2-yl)-3-fluormethyl-1-methyl-pyrazol-4-yl-carboxamid ,
N-(3',4',5'-Trifluorbiphenyl-2-yl)-3-(chlorfluormethyl)-1-methyl-pyrazol-4-ylcarboxamid,
N-(3',4',5'-Trifluorbiphenyl-2-yl)-3-difluormethyl-1-methyl-pyrazol-4-yl-carboxamid,
N-(3',4',5'-Trifluorbiphenyl-2-yl)-3-difluormethyl-5-fluoro-1-methyl-pyrazol-4-yl-carboxamid,
N-(3',4',5'-Trifluorbiphenyl-2-yl)-5-chlor-3-difluormethyl-1-methyl-pyrazol-4-yl-carboxamid,
N-(3',4',5'-Trifluorbiphenyl-2-yl)-3-(chlordifluormethyl)-1-methyl-pyrazol-4-yl-carboxamid,
N-(3',4',5'-Trifluorbiphenyl-2-yl)-1-methyl-3-trifluormethyl-pyrazol-4-yl-carboxamid,
N-(3',4',5'-Trifluorbiphenyl-2-yl)-5-fluor-1-methyl-3-trifluormethyl-pyrazol-4-yl-carboxamid,
N-(3',4',5'-Trifluorbiphenyl-2-yl)-5-chlor-1-methyl-3-trifluormethyl-pyrazol-4-yl-carboxamid,
N-(2',4',5'-Trifluorbiphenyl-2-yl)-1,3-dimethyl-pyrazol-4-yl-carboxamid,
N-(2',4',5'-Trifluorbiphenyl-2-yl)-1,3-dimethyl-5-fluor-pyrazol-4-yl-carboxamid,
N-(2',4',5'-Trifluorbiphenyl-2-yl)-5-chlor-1,3-dimethyl-pyrazol-4-yl-carboxamid,
N-(2',4',5'-Trifluorbiphenyl-2-yl)-3-fluormethyl-1-methyl-pyrazol-4-yl-carboxamid,
N-(2',4',5'-Trifluorbiphenyl-2-yl)-3-(chlorfluormethyl)-1-methyl-pyrazol-4-yl-carboxamid,
N-(2',4',5'-Trifluorbiphenyl-2-yl)-3-difluormethyl-1-methyl-pyrazol-4-yl-carboxamid,
N-(2',4',5'-Trifluorbiphenyl-2-yl)-3-difluormethyl-5-fluor-1-methylpyrazol-4-yl-carboxamid,
N-(2',4',5'-Trifluorbiphenyl-2-yl)-5-chlor-3-difluormethyl-1-methyl-pyrazol-4-yl-carboxamid,
N-(2',4',5'-Trifluorbiphenyl-2-yl)-3-(chlordifluormethyl)-1-methyl-pyrazol-4-yl-carboxamid,
N-(2',4',5'-Trifluorbiphenyl-2-yl)-1-methyl-3-trifluormethyl-pyrazol-4-yl-carboxamid, N-(2',4',5'-Trifluorbiphenyl-2-yl)-5-fluor-1-methyl-3-trifluormethyl-pyrazol-4-yl-carboxamid,
N-(2',4',5'-Trifluorbiphenyl-2-yl)-5-chlor-1-methyl-3-trifluormethyl-pyrazol-4-yl-carboxamid,
N-(3',4'-Dichlor-3-fluorbiphenyl-2-yl)-1-methyl-3-trifluormethyl-1H-pyrazol-4-yl-carboxamid,
N-(3',4'-Dichlor-3-fluorbiphenyl-2-yl)-1-methyl-3-difluormethyl-1 H-pyrazol-4-yl-carboxamid,
N-(3',4'-Difluor-3-fluorbiphenyl-2-yl)-1-methyl-3-trifluormethyl-1H-pyrazol-4-carboxamid,
N-(3',4'-Difluor-3-fluorbiphenyl-2-yl)-1-methyl-3-difluormethyl-1H-pyrazol-4-yl-carboxamid,
N-(3'-Chlor-4'-fluor-3-fluorbiphenyl-2-yl)-1-methyl-3-difluormethyl-1H-pyrazol-4-yl-carboxamid,
N-(3',4'-Dichlor-4-fluorbiphenyl-2-yl)-1-methyl-3-trifluormethyl-1H-pyrazol-4-yl-carboxamid,
N-(3',4'-Difluor-4-fluorbiphenyl-2-yl)-1-methyl-3-trifluormethyl-1H-pyrazol-4-yl-carboxamid,
N-(3',4'-Dichlor-4-fluorbiphenyl-2-yl)-1-methyl-3-difluormethyl-1H-pyrazol-4-yl-carboxamid,
N-(3',4'-Difluor-4-fluorbiphenyl-2-yl)-1-methyl-3-difluormethyl-1H-pyrazol-4-yl-carboxamid,
N-(3'-Chlor-4'-fluor-4-fluorbiphenyl-2-yl)-1-methyl-3-difluormethyl-1H-pyrazol-4-yl-carboxamid,
N-(3',4'-Dichlor-5-fluorbiphenyl-2-yl)-1-methyl-3-trifluormethyl-1H-pyrazol-4-yl-carboxamid,
N-(3',4'-Difluor-5-fluorbiphenyl-2-yl)-1-methyl-3-trifluormethyl-1H-pyrazol-4-yl-carboxamid,
N-(3',4'-Dichlor-5-fluorbiphenyl-2-yl)-1-methyl-3-difluormethyl-1H-pyrazol-4-yl-carboxamid,
N-(3',4'-Difluor-5-fluorbiphenyl-2-yl)-1-methyl-3-difluormethyl-1H-pyrazole-4-yl-carboxamid,
N-(3',4'-Dichlor-5-fluorbiphenyl-2-yl)-1,3-dimethyl-1H-pyrazol-4-yl-carboxamid,
N-(3'-Chlor-4'-fluor-5-fluorbiphenyl-2-yl)-1-methyl-3-difluormethyl-1H-pyrazole-4-yl-carboxamid,
N-(4'-Fluor-4-fluorbiphenyl-2-yl)-1-methyl-3-trifluormethyl-1H-pyrazol-4-yl-carboxamid,
N-(4'-Fluor-5-fluorbiphenyl-2-yl)-1-methyl-3-trifluormethyl-1H-pyrazole-4-yl-carboxamid,
N-(4'-Chlor-5-fluorbiphenyl-2-yl)-1-methyl-3-trifluormethyl-1H-pyrazol-4-yl-carboxamid,
N-(4'-Methyl-5-fluorbiphenyl-2-yl)-1-methyl-3-trifluormethyl-1H-pyrazol-4-yl-carboxamid,
N-(4'-Fluor-5-fluorbiphenyl-2-yl)-1,3-dimethyl-1H-pyrazol-4-yl-carboxamid,
N-(4'-Chlor-5-fluorbiphenyl-2-yl)-1,3-dimethyl-1H-pyrazol-4-yl-carboxamid,
N-(4'-Methyl-5-fluorbiphenyl-2-yl)-1,3-dimethyl-1H-pyrazol-4-yl-carboxamid,
N-(4'-Fluor-6-fluorbiphenyl-2-yl)-1-methyl-3-trifluormethyl-1H-pyrazol-4-yl-carboxamid,
N-[2-(1,1,2,3,3,3-Hexafluorpropoxy)-phenyl]-3-difluormethyl-1-methyl-1H-pyrazol-4-yl-carboxamid,
N-[4'-(Trifluormethylthio)-biphenyl-2-yl]-3-difluormethyl-1-methyl-1H-pyrazol-4-yl-carboxamid,
N-[4'-(Trifluormethylthio)-biphenyl-2-yl]-1-methyl-3-trifluormethyl-1-methyl-1H-pyrazol-4-yl-carboxamid,
3-(Difluormethyl)-1-methyl-N-[1,2,3,4-tetrahydro-9-(1-methylethyl)-1,4-methanonaphthalen-5-yl]-1H-pyrazol-4-yl-carboxamid,
N-(3'-Chlor-5-fluorbiphenyl-2-yl)- 3-(difluormethyl)-1-methylpyrazol-4-yl-carboxamid,
N-(4'-Chlor-5-fluorbiphenyl-2-yl)- 3-(difluormethyl)-1-methylpyrazol-4-yl-carboxamid,
N-(4'-Chlorbiphenyl-2-yl)-3-(difluormethyl)-1-methylpyrazol-4-yl-carboxamid,
N-(4'-Brombiphenyl-2-yl)-3-(difluormethyl)-1-methylpyrazol-4-yl-carboxamid,
N-(4'-Jodbiphenyl-2-yl)-3-(difluormethyl)-1-methylpyrazol-4-yl-carboxamid,
N-(3',5'-Difluorbiphenyl-2-yl)-3-(difluormethyl)-1-methylpyrazol-4-yl-carboxamid,
N-(2-Chlor-4-fluor-phenyl)- 3-(difluormethyl)-1-methylpyrazol-4-yl-carboxamid,
N-(2-Brom-4-fluor-phenyl)- 3-(difluormethyl)-1-methylpyrazol-4-yl-carboxamid,
N-(2-Jod-4-fluor-phenyl)- 3-(difluormethyl)-1-methylpyrazol-4-yl-carboxamid und
N-[2-(1,3-Dimethylbutyl)-phenyl]-1,3-dimethyl-5-fluor-1H-pyrazol-4-yl-carboxamid.

Besonders bevorzugt sind dabei diejenigen Carboxamide (I), bei denen

Ar für einen ein- bis dreifach substituierten Pyridyl- oder Pyrazolylring, wobei die Substituenten unabhängig voneinander ausgewählt sind aus Halogen, C₁-C₄-Alkyl und C₁-C₄-Halogenalkyl, steht. Ganz besonders bevorzugt sind diejenigen Carboxamide (I), bei denen Ar für einen zwei- oder dreifach substituierten Pyrazolylring, wobei die Substituenten unabhängig voneinander ausgewählt sind aus Halogen, C₁-C₄-Alkyl und C₁-C₄-Halogenalkyl, insbesondere Fluor, Chlor, Methyl, Difluormethyl und Trifluormethyl, steht.

Die Reaktionsführung erfolgt erfindungsgemäß ohne Hilfsbase in einem organischen Lösungsmittel, das weitgehend wasserfrei ist. Unter einem geringen Wassergehalt werden dabei etwa 0,5 g bis 5 g Wasser pro mol an eingesetztem Säurechlorid (II) verstanden. Größere Wassermengen sind zu vermeiden, da das Wasser zu einem erhöhten Verbrauch an Einsatzstoffen führen würde.

Brauchbare Lösungsmittel sind beispielsweise aromatische Kohlenwasserstoffe wie Toluol, o-,m-,p-Xylol, Mesitylen, Ethylbenzol und Chlorbenzol, halogenierte aliphatische Kohlenwasserstoffe wie Tetrachlorethan und Dichlorethylen, Ether wie Methyl-tert.-butylethyl, Tetrahydrofuran und Dioxan oder Mischungen der genannten Solventien. Besonders bevorzugte Lösungsmittel sind die aromatischen Kohlenwasserstoffe, insbesondere Toluol und o-,m-,p-Xylol.

Erfindungsgemäß legt man das Säurechlorid (II) vor, stellt den gewünschten Druck ein und dosiert das Arylamin (III) zu. Unter Zudosierung versteht man dabei sowohl die portionsweise als auch kontinuierliche Zugabe von (III)
a) auf die Oberläche der Lösung von (II) oder
b) direkt in die Lösung von (II), als "getauchte Reaktionsführung" bezeichnet.

Der Druck wird in der Regel so gewählt, dass die Reaktionsmischung siedet.

Normalerweise arbeitet man bei einem Druck zwischen 0 und 700 mbar und einer Reaktionstemperatur von 20 bis 120°C, vorzugsweise bei 200 bis 600 mbar und 70 bis 100°C, insbesondere bei 350 bis 450 mbar und 80 bis 90°C.

Säurechlorid (II) und Arylamin (III) werden etwa äquimolar oder eine der Komponenten in einem leichten Überschuß bis 10 mol% eingesetzt. Das Molverhältnis von (III) zu (II) liegt damit in der Regel bei 0,9 : 1 bis 1,1 : 1, vorzugsweise bei etwa 1,0 bis 1,1.

Die Zudosierung von (III), vorzugsweise gelöst in dem organischen Lösungsmittel, in dem auch (II) vorgelegt worden ist, erfolgt üblicherweise im Verlauf von 0,5 bis 20 Stunden, insbesondere 2 bis 10 Stunden, besonders bevorzugt 3 bis 5 Stunden.

Die Freisetzung des Carboxamids (I) aus der Reaktionsmischung erfolgt vorzugsweise durch direkte Kristallisation oder durch Behandlung der Reaktionsmischung mit einer geeigneten Base und anschließender Kristallisation, z.B. bei (-20) bis 20°C.

Als Base eignen sich hierfür Alkalimetallhydroxide wie Natrium- und Kaliumhydroxid, Alkalimetallcarbonate wie Natrium- und Kaliumcarbonat, Alkalimetallhydrogencarbonate wie Natium- und Kaliumhydrogencarbonat, Alkalimetallphosphate wie Natrium- und Kaliumphosphat, Alkalimetallhydrogenphosphate wie Natrium- und Kaliumhydrogenphosphat, Alkalimetaldilhydrogenphosphate wie Natrium- und Kaliumdihydrogenphosphat, sowie Stickstoffbasen wie Ammoniak.

Besonders bevorzugt sind die Alkalimetallhydroxide wie Natrium- und Kaliumhydroxid, Alkalimetallcarbonate wie Natrium- und Kaliumcarbonat sowie die Alkalimetallhydrogencarbonate wie Natium- und Kaliumhydrogencarbonat.

Die Base kann in fester Form oder in Form ihrer handelsüblichen wässrigen Lösungen eingesetzt werden. Vorzugsweise verwendet man eine 1 bis 20 Gew.%ige wässrige Lösung, wobei die Menge so zu bemessen ist, dass der pH-Wert der Lösung bei 3 bis 12, vorzugsweise 7 bis 10, liegt.

Das kristalline Wertprodukt kann schließlich mittels üblicher Methoden, z.B. Filtration, abgetrennt werden.

Die Verfahrensprodukte (I) sind wertvolle Wirkstoffe im Pflanzenschutz.

### Ausführungsbeispiele:

### Beispiel 1

### Synthese von 3-Difluormethyl-1-methyl-1H-pyrazol-4-carbonsäure (3', 4', 5'-trifluorbiphenyl-2-yl)-amid

100,0 g (0,504 mol, 98 %ig rein) 3-Difluormethyl-1-methyl-1H-pyrazol-4-carbonsäurechlorid wurde bei 25°C in 257,2 g Toluol gelöst. Die Lösung wurde auf 400 mbar evakuiert und auf 85°C erwärmt. Anschließend wurden innerhalb von 3 Stunden 492,8 g (0,499 mol, 23 %ige) toluolische 3',4',5'-Trifluor-biphenyl-2-yl-amin-Lösung zudosiert, wonach man noch 1 Stunde nachrührte. Nach Belüftung und Abkühlung mit einer Rampe von 10°C/Std. auf 25°C wurde über Nacht gerührt. Anschließend wurde auf 0°C abgekühlt, die festen Bestandteile abfiltriert, mit kaltem Toluol gewaschen und bei 80°C unter reduziertem Druck getrocknet. Die Ausbeute (ohne weitere Bearbeitung der Mutterlauge) lag bei 177,7 g (92 %).

### Beispiel1a (Vergleichsversuch)

Synthese von 3-Difluormethyl-1-methyl-1H-pyrazol-4-carbonsäure (3',4',5'-trifluorbiphenyl-2-yl)-amid (analoge Reaktionsführung zu Beispiel 1 aus JP-A 2001/172276, S. 10)

19,0 g (0,085 mol, 99,8 %ig rein) 3',4',5'-Trifluor-biphenyl-2-yl amin wurden in 400,0 g Toluol gelöst. Innerhalb von 1 Min. wurden 17,7 g (0,089 mol, 98,1 %ig) 3-Difluormethyl-1-methyl-1H-pyrazol-4-carbonsäurechlorid bei 25°C zugegeben. Anschließend wurde die Reaktionsmischung auf 72 mbar evakuiert und 3 Stunden lang auf 40°C erhitzt. Nach 15 Min. bildete sich ein weißer Feststoff, der im weiteren Verlauf zu einer viskosen, gelartigen Suspension überging. Nach Abkühlung auf 0°C wurde über eine Glasfritte filtriert (sehr langsam, Verstopfungen) und der Filterkuchen mit kaltem Toluol gewaschen. Der Rückstand wurde bei reduziertem Druck getrocknet und lieferte 15,0 g
eines Gemisches aus 3',4',5'-Trifluor-biphenyl-2-yl amin hydrochlorid (40 Gew.%) und 3-Difluormethyl-1-methyl-1H-pyrazol-4-carbonsäure (3',4',5'-trifluor-biphenyl-2-yl)-amid (48 Gew.%). Die Mutterlauge (375,0 g) enthielt 2,8 Gew.% 3-Difluormethyl-1-methyl-1H-pyrazol-4-carbonsäure (3',4',5'-trifluor-biphenyl-2-yl)-amid. Rein rechnerisch betrug die Ausbeute somit ca. 54 %.

### Beispiel 2

### Synthese von 3-Difluormethyl-1-methyl-1H-pyrazol-4-carbonsäure (3',5'-difluorbiphenyl-2-yl)-amid

38,9 g (0,196 mol, 98 %ig rein) 3-Difluormethyl-1-methyl-1H-pyrazol-4-carbonsäurechlorid wurde bei 25°C in 100,0 g Toluol gelöst. Die Lösung wurde auf 400 mbar evakuiert und auf 85°C erwärmt. Anschließend wurden innerhalb von 1,5 Stunden 173,0 g (0,194 mol, 23 %ig) toluolische 3',4'-Difluor-biphenyl-2-yl amin-Lösung zudosiert und die Reaktionsmischung 1 Stunde nachgerührt. Nach Belüftung und Abkühlung auf Raumtemperatur wurde unter reduziertem Druck auf ca. 100 ml Volumen eingeengt. Der Feststoffanteil wurde abfiltriert, mit n-Hexan gewaschen und bei 85°C unter reduziertem Druck getrocknet. Die Ausbeute (ohne weitere Bearbeitung der Mutterlauge) lag bei 46,5 g (66 %).

### Beispiel 3

### Synthese von 1,3-Dimethyl-1H-pyrazol-4-carbonsäure (3', 4', 5'-trifluor-biphenyl-2-yl)-amid

79,1 g (0,494 mol, 99 %ig rein) 1,3-Dimethyl-1H-pyrazol-4-carbonsäurechlorid wurde bei 25°C in 257,2 g Toluol gelöst. Die Lösung wurde auf 400 mbar evakuiert und auf 85°C erwärmt. Anschließend wurden innerhalb von 3 Stunden 483,0 g (0,489 mol, 23 %ig) toluolische 3',4',5'-Trifluor-biphenyl-2-yl amin-Lösung dosiert und die Reaktionsmischung 1 Stunde nachgerührt. Nach Belüftung und Abkühlung auf 70°C wurde mit einer Kühlrampe von 5°C/Std. auf 20°C gekühlt und über Nacht gerührt. Anschließend wurde auf 0°C abgekühlt, der Feststoffanteil abfiltriert, mit kaltem Toluol gewaschen und bei 80°C unter reduziertem Druck getrocknet. Die Ausbeute (ohne weitere Bearbeitung der Mutterlauge) lag bei 155,4 g (92 %).

### Beispiel 4

### Synthese von 3-Difluormethyl-1-methyl-1H-pyrazol-4-carbonsäure (2-chlor-phenyl)-amid

80,0 g (0,403 mol, 98 %ig rein) 3-Difluormethyl-1-methyl-1H-pyrazol-4-carbonsäurechlorid wurden bei 25°C in 257,2 g Toluol gelöst. Die Lösung wurde auf 400 mbar evakuiert und auf 85°C erwärmt. Anschließend wurden innerhalb von 3 Stunden 221,3 g (0,399 mol, 23 %ig) toluolische 2-Chlor-anilin -Lösung zudosiert und die Reaktionsmischung 1 Stunde nachgerührt. Nach Belüftung und Abkühlung mit einer Rampe von 10°C/Std. auf 20°C wurde über Nacht gerührt. Anschließend wurde auf 0°C abgekühlt, der Feststoffanteil abfiltriert, mit kaltem Toluol gewaschen und bei 80°C unter reduziertem Druck getrocknet. Die Ausbeute (ohne weitere Bearbeitung der Mutterlauge) lag bei 105 g (92 %).

### Beispiel 5

### Synthese von 3-Difluormethyl-1-methyl-1H-pyrazol-4-carbonsäure (3', 4'-dichlor-5-fluor-biphenyl-2-yl)-amid

5,6 g (0,029 mol, 98 %ig rein) 3-Difluormethyl-1-methyl-1H-pyrazol-4-carbonsäurechlorid wurden bei 25°C in 10,4 g Toluol gelöst. Die Lösung wurde auf 400 mbar evakuiert und auf 85°C erwärmt. Anschließend wurden innerhalb von 5 Minuten 7,8 g (0,030 mol, ca. 92 %ig) 3',4'-Dichlor-5-fluor-biphenyl-2-yl-amin, gelöst in 28 g Toluol, zudosiert und die Reaktionsmischung 1 Stunde nachgerührt. Nach Belüftung und Abkühlung auf 25°C über Nacht wurde weiter auf 0°C abgekühlt, der Feststoffanteil abfiltriert, mit kaltem Toluol gewaschen und bei 80°C unter reduziertem Druck getrocknet. Die Ausbeute (ohne weitere Bearbeitung der Mutterlauge) lag bei 8,1 g (71 %).

### Beispiel 6

### Synthese von 3-Difluormethyl-1-methyl-1H-pyrazol-4-carbonsäure (2-bicyclopropyl-2-yl-phenyl)-amid

16,7 g (0,086 mol, 98 %ig rein) 3-Difluormethyl-1-methyl-1H-pyrazol-4-carbonsäurechlorid wurden bei 25°C in 48,0 g Toluol gelöst. Die Lösung wurde auf 400 mbar evakuiert und auf 85°C erwärmt. Anschließend wurden innerhalb von 45 Min. 15,0 g (0,087 mol) 2-Bicyclopropyl-2-yl-phenylamin, gelöst in 51,6 g Toluol, zudosiert und die Reaktionsmischung 1 Std. nachgerührt. Nach Belüftung und Abkühlung auf 25°C wurde über Nacht gerührt. Anschließend wurde das Gemisch unter reduziertem Druck eingeengt und getrocknet. Die Ausbeute lag bei 27,3 g (96 %).

### Beispiel 7

### Synthese von 3-Difluormethyl-1-methyl-1H-pyrazol-4-carbonsäure (9-isopropyl-1,2,3,4-tetrahydro-1,4-methano-naphthalen-5-yl)-amid

15,1 g (0,0752 mol, 96,5%ig rein) 3-Difluormethyl-1-methyl-1H-pyrazol-4-carbonsäurechlorid wurden bei 25°C in 100 ml Toluol gelöst. Die Lösung wurde auf 350 mbar evakuiert und auf 85°C erwärmt. Anschließend wurden innerhalb von 60 min 20 g (0,074 mol, 75 %; syn/anti-Isomerengemisch 65:10) 9-Isopropyl-1,2,3,4-tetrahydro-1,4-methano-naphthalen-5-ylamin, gelöst in 100ml Toluol, zudosiert und die Reaktionsmischung 1 Stunde nachgerührt. Nach Belüftung und Abkühlung auf 25°C wurde über Nacht gerührt. Anschließend wurde das Gemisch unter reduziertem Druck eingeengt und getrocknet. Die Ausbeute lag bei 31,3 g; gemäß 1H-NMR 70%ig (82 %).

### Beispiel 8

### Synthese von 2-Chlor-N-(4'-chlor-biphenyl-2-yl)nicotinamid

100,0 g (0,557 mol, 98 %ig rein) 2-chlornicotinsäurechlorid wurden bei 25°C in 80,0 g Toluol gelöst. Die Lösung wurde auf 200 mbar evakuiert und auf 95°C erwärmt. Anschließend wurden innerhalb von 2,5 Stunden 396,8 g (0,541 mol, 28 %ig) xylolische 4'Chlor-biphenyl-2-yl-amin-Lösung dosiert und die Reaktionsmischung 1 Stunde nachgerührt. Nach Belüftung und Abkühlung auf 87°C wurde mit 1 g 2-Chlor-N-(4'-chlor-biphenyl-2-yl)nicotinamid angeimpft und die Temperatur für 1 Stunde gehalten. Anschließend wurde mit einer Rampe von 5°C/Std. auf 25°C abgekühlt. Nach weiterer Kühlung auf 10-15°C wurde der Feststoffanteil abfiltriert, mit kaltem Xylol gewaschen und bei 80°C unter reduziertem Druck getrocknet. Die Ausbeute (ohne weitere Bearbeitung der Mutterlauge) lag bei 166,4 g (73 %). Das HPLC zeigt das gewünschte Produkt und das doppelt acylierte Produkt im Verhältnis 85:15 Flächen%.

## Patentansprüche

1. Verfahren zur Herstellung von Arylcarboxamiden der Formel (I) 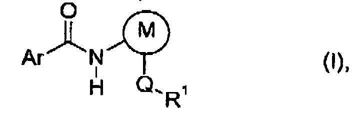 wobei die Substituenten folgende Bedeutungen haben:
Ar ein- bis dreifach substituierter Phenyl-, Pyridyl- oder Pyrazolylring, wobei die Substituenten unabhängig voneinander ausgewählt sind aus Halogen, C₁-C₄-Alkyl und C₁-C₄-Halogenalkyl;
M Thienyl oder Phenyl, das einen Halogensubstituenten tragen kann;
Q eine direkte Bindung, Cyclopropylen, ein annellierter Bicyclo[2.2.1]heptan-oder Bicyclo[2.2.1]hepten-Ring;
R¹ Wasserstoff, Halogen, C₁-C₆-Alkyl, C₁-C₄-Alkoxy, C₁-C₄-Halogenalkoxy, einbis dreifach substituiertes Phenyl, wobei die Substituenten unabhängig voneinander ausgewählt sind aus Halogen und Trifluormethylthio, oder Cyclopropyl;
durch Umsetzung eines Säurechlorids der Formel (II) 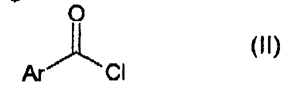 mit einem Arylamin (III) 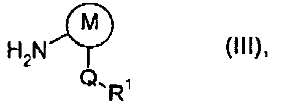 in einem geeigneten nicht-wässrigen Lösungsmittel,
**dadurch gekennzeichnet, dass** man in Abwesenheit einer Hilfsbase
a) das Säurechlorid (II) vorlegt,
b) einen Druck von 0 bis 700 mbar einstellt,
c) das Arylamin (III) zudosiert, so dass das Molverhältnis von (II) zu (III) 0,9 : 1 bis 1,1 : 1 beträgt und
d) das Wertprodukt isoliert.

2. Verfahren nach Anspruch 1, wobei Ar ein Phenyl-, Pyridyl- oder Pyrazolylring 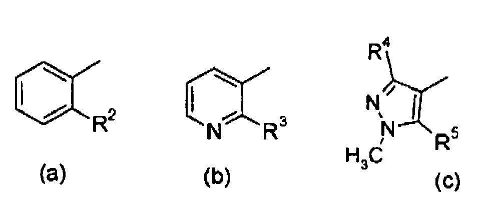 ist und
R² für Halogen, Methyl oder Trifluormethyl;
R³ für Halogen;
R⁴ für C₁-C₄-Alkyl oder C₁-C₄-Haloalkyl und
R⁵ für Wasserstoff oder Halogen steht.

3. Verfahren nach Anspruch 1, wobei M für Phenyl, Q für Cyclopropylene und R¹ für Cyclopropyl steht.

4. Verfahren nach Anspruch 1, wobei M für Phenyl, Q für eine Bindung und R¹ für Isopropoxy oder 1- bis 3-fach substituiertes Phenyl, wobei die Substituenten unabhängig voneinander ausgewählt sind aus Halogen und Trifluormethylthio, steht.

5. Verfahren nach Anspruch 1, wobei M für durch ein Halogen substituiertes Phenyl, Q für eine Bindung und R¹ für Wasserstoff oder 1- bis 3-fach durch Halogen substituiertes Phenyl steht.

6. Verfahren nach Anspruch 1, wobei das Arylcarboxamid Benodanil, Bixafen, Boscalid, Flutolanil, Mepronil, Penthiopyrad,
N-(2-Bicyclopropyl-2-yl-phenyl)-3-difluormethyl-1-methylpyrazol-4-yl-carboxamid,
N-(3',4',5'-Trifluorbiphenyl-2-yl)-1,3-dimethyl-pyrazol-4-yl-carboxamid,
N-(3',4',5'-Trifluorbiphenyl-2-yl)-1,3-dimethyl-5-fluor-pyrazol-4-yl-carboxamid,
N-(3',4',5'-Trifluorbiphenyl-2-yl)-5-chlor-1,3-dimethyl-pyrazol-4-yl-carboxamid,
N-(3',4',5'-Trifluorbiphenyl-2-yl)-3-fluormethyl-1-methyl-pyrazol-4-yl-carboxamid,
N-(3',4',5'-Trifluorbiphenyl-2-yl)-3-(chlorfluormethyl)-1-methyl-pyrazol-4-ylcarboxamid,
N-(3',4',5'-Trifluorbiphenyl-2-yl)-3-difluormethyl-1-methyl-pyrazol-4-yl-carboxamid,
N-(3',4',5'-Trifluorbiphenyl-2-yl)-3-difluormethyl-5-fluoro-1-methyl-pyrazol-4-yl-carboxamid,
N-(3',4', 5'-Trifluorbiphenyl-2-yl)-5-chlor-3-difluormethyl-1-methyl-pyrazol-4-yl-carboxamid,
N-(3',4',5'-Trifluorbiphenyl-2-yl)-3-(chlordifluormethyl)-1-methyl-pyrazol-4-yl-carboxamid,
N-(3',4',5'-Trifluorbiphenyl-2-yl)-1-methyl-3-trifluormethyl-pyrazol-4-yl-carboxamid,
N-(3',4',5'-Trifluorbiphenyl-2-yl)-5-fluor-1-methyl-3-trifluormethyl-pyrazol-4-yl-carboxamid,
N-(3',4',5'-Trifluorbiphenyl-2-yl)-5-chlor-1-methyl-3-trifluormethyl-pyrazol-4-yl-carboxamid,
N-(2',4',5'-Trifluorbiphenyl-2-yl)-1,3-dimethyl-pyrazol-4-yl-carboxamid,
N-(2',4',5'-Trifluorbiphenyl-2-yl)-1,3-dimethyl-5-fluor-pyrazol-4-yl-carboxamid,
N-(2',4',5'-Trifluorbiphenyl-2-yl)-5-chlor-1,3-dimethyl-pyrazol-4-yl-carboxamid,
N-(2',4',5'-Trifluorbiphenyl-2-yl)-3-fluormethyl-1-methyl-pyrazol-4-yl-carboxamid,
N-(2',4',5'-Trifluorbiphenyl-2-yl)-3-(chlorfluormethyl)-1-methyl-pyrazol-4-yl-carboxamid,
N-(2',4',5'-Trifluorbiphenyl-2-yl)-3-difluormethyl-1-methyl-pyrazol-4-yl-carboxamid,
N-(2',4',5'-Trifluorbiphenyl-2-yl)-3-difluormethyl-5-fluor-1-methylpyrazol-4-yl-carboxamid,
N-(2',4',5'-Trifluorbiphenyl-2-yl)-5-chlor-3-difluormethyl-1-methyl-pyrazol-4-yl-carboxamid,
N-(2',4',5'-Trifluorbiphenyl-2-yl)-3-(chlordifluormethyl)-1-methyl-pyrazol-4-yl-carboxamid,
N-(2',4',5'-Trifluorbiphenyl-2-yl)-1-methyl-3-trifluormethyl-pyrazol-4-yl-carboxamid,
N-(2',4',5'-Trifluorbiphenyl-2-yl)-5-fluor-1-methyl-3-trifluormethyl-pyrazol-4-yl-carboxamid,
N-(2',4',5'-Trifluorbiphenyl-2-yl)-5-chlor-1-methyl-3-trifluormethyl-pyrazol-4-yl-carboxamid,
N-(3',4'-Dichlor-3-fluorbiphenyl-2-yl)-1-methyl-3-trifluormethyl-1H-pyrazol-4-yl-carboxamid,
N-(3',4'-Dichlor-3-fluorbiphenyl-2-yl)-1-methyl-3-difluormethyl-1H-pyrazol-4-yl-carboxamid,
N-(3',4'-Difluor-3-fluorbiphenyl-2-yl)-1-methyl-3-trifluormethyl-1H-pyrazol-4-carboxamid,
N-(3',4'-Difluor-3-fluorbiphenyl-2-yl)-1-methyl-3-difluormethyl-1H-pyrazol-4-yl-carboxamid,
N-(3'-Chlor-4'-fluor-3-fluorbiphenyl-2-yl)-1-methyl-3-difluormethyl-1H-pyrazol-4-yl-carboxamid,
N-(3',4'-Dichlor-4-fluorbiphenyl-2-yl)-1-methyl-3-trifluormethyl-1H-pyrazol-4-yl-carboxamid,
N-(3',4'-Difluor-4-fluorbiphenyl-2-yl)-1-methyl-3-trifluormethyl-1H-pyrazol-4-yl-carboxamid,
N-(3',4'-Dichlor-4-fluorbiphenyl-2-yl)-1-methyl-3-difluormethyl-1H-pyrazol-4-yl-carboxamid,
N-(3',4'-Difiuor-4-fluorbiphenyl-2-yl)-1-methyl-3-difluormethyl-1H-pyrazol-4-yl-carboxamid,
N-(3'-Chlor-4'-fluor-4-fluorbiphenyl-2-yl)-1-methyl-3-difluormethyl-1H-pyrazol-4-yl-carboxamid,
N-(3',4'-Dichlor-5-fluorbiphenyl-2-yl)-1-methyl-3-trifluormethyl-1H-pyrazol-4-yl-carboxamid,
N-(3',4'-Difluor-5-fluorbiphenyl-2-yl)-1-methyl-3-trifluormethyl-1H-pyrazol-4-yl-carboxamid,
N-(3',4'-Dichlor-5-fluorbiphenyl-2-yl)-1-methyl-3-difluormethyl-1H-pyrazol-4-yl-carboxamid,
N-(3',4'-Difluor-5-fluorbiphenyl-2-yl)-1-methyl-3-difluormethyl-1H-pyrazole-4-yl-carboxamid.
N-(3',4'-Dichlor-5-fluorbiphenyl-2-yl)-1,3-dimethyl-1H-pyrazol-4-yl-carboxamid,
N-(3'-Chlor-4'-fluor-5-fluorbiphenyl-2-yl)-1-methyl-3-difluormethyl-1H-pyrazole-4-yl-carboxamid,
N-(4'-Fluor-4-fluorbiphenyl-2-yl)-1-methyl-3-trifluormethyl-1H-pyrazol-4-yl-carboxamid,
N-(4'-Fluor-5-fluorbiphenyl-2-yl)-1-methyl-3-trifluormethyl-1H-pyrazole-4-yl-carboxamid,
N-(4'-Chlor-5-fluorbiphenyl-2-yl)-1-methyl-3-trifluormethyl-1H-pyrazol-4-yl-carboxamid,
N-(4'-Methyl-5-fluorbiphenyl-2-yl)-1-methyl-3-trifluormethyl-1H-pyrazol-4-yl-carboxamid,
N-(4'-Fluor-5-fluorbiphenyl-2-yl)-1,3-dimethyl-1H-pyrazol-4-yl-carboxamid,
N-(4'-Chlor-5-fluorbiphenyl-2-yl)-1,3-dimethyl-1H-pyrazol-4-yl-carboxamid,
N-(4'-Methyl-5-fluorbiphenyl-2-yl)-1,3-dimethyl-1H-pyrazol-4-yl-carboxamid,
N-(4'-Fluor-6-fluorbiphenyl-2-yl)-1-methyl-3-trifluormethyl-1H-pyrazol-4-yl-carboxamid,
N-[2-(1,1,2,3,3,3-Hexafluorpropoxy)-phenyl]-3-difluormethyl-1-methyl-1H-pyrazol-4-yl-carboxamid,
N-[4'-(Trifluormethylthio)-biphenyl-2-yl]-3-difluormethyl-1-methyl-1H-pyrazol-4-yl-carboxamid,
N-[4'-(Trifluormethylthio)-biphenyl-2-yl]-1-methyl-3-trifluormethyl-1-methyl-1H-pyrazol-4-yl-carboxamid,
3-(Difluormethyl)-1-methyl-N-[1,2,3,4-tetrahydro-9-(1-methylethyl)-1,4-methanonaphthalen-5-yl]-1H-pyrazol-4-yl-carboxamid,
N-(3'-Chlor-5-fluorbiphenyl-2-yl)- 3-(difluormethyl)-1-methylpyrazol-4-yl-carboxamid,
N-(4'-Chlor-5-fluorbiphenyl-2-yl)-3-(difluormethyl)-1-methylpyrazol-4-yl-carboxamid,
N-(4'-Chlorbiphenyl-2-yl)-3-(difluormethyl)-1-methylpyrazol-4-yl-carboxamid,
N-(4'-Brombiphenyl-2-yl)-3-(difluormethyl)-1-methylpyrazol-4-yl-carboxamid,
N-(4'-Jodbiphenyl-2-yl)-3-(difluormethyl)-1-methylpyrazol-4-yl-carboxamid.
N-(3',5'-Difluorbiphenyl-2-yl)-3-(difluormethyl)-1-methylpyrazol-4-yl-carboxamid,
N-(2-Chlor-4-fluor-phenyl)- 3-(difluormethyl)-1-methylpyrazol-4-yl-carboxamid,
N-(2-Brom-4-fluor-phenyl)- 3-(difluormethyl)-1-methylpyrazol-4-yl-carboxamid,
N-(2-Jod-4-fluor-phenyl)- 3-(difluormethyl)-1-methylpyrazol-4-yl-carboxamid oder
N-[2-(1,3-Dimethylbutyl)-phenyl]-1,3-dimethyl-5-fluor-1H-pyrazol-4-yl-carboxamid ist.

7. Verfahren nach Anspruch 1, **dadurch gekennzeichnet, dass** man die Umsetzung von II mit III bei einem Druck von 200 bis 600 mbar vornimmt.

8. Verfahren nach Anspruch 1, **dadurch gekennzeichnet, dass** man die Umsetzung von II mit III bei 20 bis 120°C vornimmt.

9. Verfahren nach einem der Ansprüche 1 bis 8, **dadurch gekennzeichnet, dass** die die Zugabe von Arylamin (III) portionsweise oder kontinuierlich auf die Oberläche der Lösung von Säurechlorid (II) oder direkt in die Lösung von Säurechlorid (II) als "getauchte Reaktionsführung" erfolgt.

## Claims

1. A process for preparing arylcarboxamides of the formula (I) 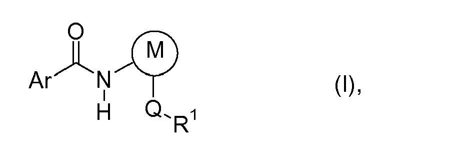 where the substituents are each defined as follows:
Ar is a mono- to trisubstituted phenyl, pyridyl or pyrazolyl ring, where the substituents are each independently selected from halogen, C₁-C₄-alkyl and C₁-C₄-haloalkyl;
M is thienyl or phenyl, which may bear a halogen substituent;
Q is a direct bond, cyclopropylene, a fused bicyclo[2.2.1]heptane or bicyclo[2.2.1]heptene ring;
R¹ is hydrogen, halogen, C₁-C₆-alkyl, C₁-C₄-alkoxy, C₁-C₄-haloalkoxy, mono- to trisubstituted phenyl, where the substituents are each independently selected from halogen and trifluoromethylthio, or cyclopropyl;
by reacting an acid chloride of the formula (II) 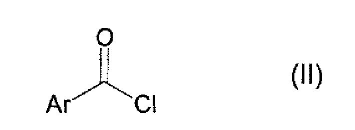 with an arylamine (III) 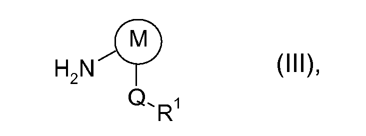 in a suitable nonaqueous solvent,
which comprises, in the absence of an auxiliary base,
a) initially charging the acid chloride (II),
b) establishing a pressure of from 0 to 700 mbar,
c) metering in the arylamine (III) such that the molar ratio of (II) to (III) is from 0.9 : 1 to 1.1 : 1 and
d) isolating the product of value.

2. The process according to claim 1, wherein Ar is a phenyl, pyridyl or pyrazolyl ring 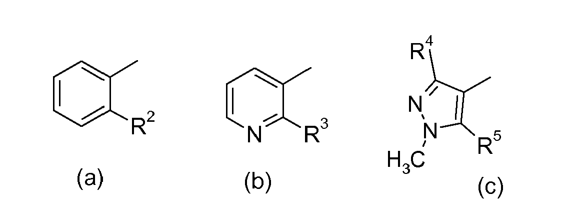 and
R² is halogen, methyl or trifluoromethyl;
R³ is halogen;
R⁴ is C₁-C₄-alkyl or C₁-C₄-haloalkyl and
R⁵ is hydrogen or halogen.

3. The process according to claim 1, wherein M is phenyl, Q is cyclopropylene and R¹ is cyclopropyl.

4. The process according to claim 1, wherein M is phenyl, Q is a bond and R¹ is isopropoxy or mono- to trisubstituted phenyl, where the substituents are each independently selected from halogen and trifluoromethylthio.

5. The process according to claim 1, wherein M is phenyl substituted by one halogen, Q is a bond and R¹ is hydrogen or mono- to tri-halogen-substituted phenyl.

6. The process according to claim 1, wherein the arylcarboxamide is benodanil, bixafen, boscalid, flutolanil, mepronil, penthiopyrad,
N-(2-bicyclopropyl-2-ylphenyl)-3-difluoromethyl-1-methylpyrazol-4-ylcarboxamide,
N-(3',4',5'-trifluorobiphenyl-2-yl)-1,3-dimethylpyrazol-4-ylcarboxamide,
N-(3',4',5'-trifluorobiphenyl-2-yl)-1,3-dimethyl-5-fluoropyrazol-4-ylcarboxamide,
N-(3',4',5'-trifluorobiphenyl-2-yl)-5-chloro-1,3-dimethylpyrazol-4-ylcarboxamide,
N-(3',4',5'-trifluorobiphenyl-2-yl)-3-fluoromethyl-1-methylpyrazol-4-ylcarboxamide,
N-(3',4',5'-trifluorobiphenyl-2-yl)-3-(chlorofluoromethyl)-1-methylpyrazol-4-ylcarboxamide,
N-(3',4',5'-trifluorobiphenyl-2-yl)-3-difluoromethyl-1-methylpyrazol-4-ylcarboxamide,
N-(3',4',5'-trifluorobiphenyl-2-yl)-3-difluoromethyl-5-fluoro-1-methylpyrazol-4-ylcarboxamide,
N-(3',4',5'-trifluorobiphenyl-2-yl)-5-chloro-3-difluoromethyl-1-methylpyrazol-4-ylcarboxamide,
N-(3',4',5'-trifluorobiphenyl-2-yl)-3-(chlorodifluoromethyl)-1-methylpyrazol-4-ylcarboxamide,
N-(3',4',5'-trifluorobiphenyl-2-yl)-1-methyl-3-trifluoromethylpyrazol-4-ylcarboxamide,
N-(3',4',5'-trifluorobiphenyl-2-yl)-5-fluoro-1-methyl-3-trifluoromethylpyrazol-4-ylcarboxamide,
N-(3',4',5'-trifluorobiphenyl-2-yl)-5-chloro-1-methyl-3-trifluoromethylpyrazol-4-ylcarboxamide,
N-(2',4',5'-trifluorobiphenyl-2-yl)-1,3-dimethylpyrazol-4-ylcarboxamide,
N-(2',4',5'-trifluorobiphenyl-2-yl)-1,3-dimethyl-5-fluoropyrazol-4-ylcarboxamide,
N-(2',4',5'-trifluorobiphenyl-2-yl)-5-chloro-1,3-dimethylpyrazol-4-ylcarboxamide,
N-(2',4',5'-trifluorobiphenyl-2-yl)-3-fluoromethyl-1-methylpyrazol-4-ylcarboxamide,
N-(2',4',5'-trifluorobiphenyl-2-yl)-3-(chlorofluoromethyl)-1-methylpyrazol-4-ylcarboxamide,
N-(2',4',5'-trifluorobiphenyl-2-yl)-3-difluoromethyl-1-methylpyrazol-4-ylcarboxamide,
N-(2',4',5'-trifluorobiphenyl-2-yl)-3-difluoromethyl-5-fluoro-1-methylpyrazol-4-ylcarboxamide,
N-(2',4',5'-trifluorobiphenyl-2-yl)-5-chloro-3-difluoromethyl-1-methylpyrazol-4-ylcarboxamide,
N-(2',4',5'-trifluorobiphenyl-2-yl)-3-(chlorodifluoromethyl)-1-methylpyrazol-4-ylcarboxamide,
N-(2',4',5'-trifluorobiphenyl-2-yl)-1-methyl-3-trifluoromethylpyrazol-4-ylcarboxamide,
N-(2',4',5'-trifluorobiphenyl-2-yl)-5-fluoro-1-methyl-3-trifluoromethylpyrazol-4-ylcarboxamide,
N-(2',4',5'-trifluorobiphenyl-2-yl)-5-chloro-1-methyl-3-trifluoromethylpyrazol-4-ylcarboxamide,
N-(3',4'-dichloro-3-fluorobiphenyl-2-yl)-1-methyl-3-trifluoromethyl-1H-pyrazol-4-ylcarboxamide,
N-(3',4'-dichloro-3-fluorobiphenyl-2-yl)-1-methyl-3-difluoromethyl-1H-pyrazol-4-ylcarboxamide,
N-(3',4'-difluoro-3-fluorobiphenyl-2-yl)-1-methyl-3-trifluoromethyl-1H-pyrazol-4-carboxamide,
N-(3',4'-difluoro-3-fluorobiphenyl-2-yl)-1-methyl-3-difluoromethyl-1H-pyrazol-4-ylcarboxamide,
N-(3'-chloro-4'-fluoro-3-fluorobiphenyl-2-yl)-1-methyl-3-difluoromethyl-1H-pyrazol-4-ylcarboxamide,
N-(3',4'-dichloro-4-fluorobiphenyl-2-yl)-1-methyl-3-trifluoromethyl-1H-pyrazol-4-ylcarboxamide,
N-(3',4'-difluoro-4-fluorobiphenyl-2-yl)-1-methyl-3-trifluoromethyl-1H-pyrazol-4-ylcarboxamide,
N-(3',4'-dichloro-4-fluorobiphenyl-2-yl)-1-methyl-3-difluoromethyl-1H-pyrazol-4-ylcarboxamide,
N-(3',4'-difluoro-4-fluorobiphenyl-2-yl)-1-methyl-3-difluoromethyl-1H-pyrazol-4-ylcarboxamide,
N-(3'-chloro-4'-fluoro-4-fluorobiphenyl-2-yl)-1-methyl-3-difluoromethyl-1H-pyrazol-4-ylcarboxamide,
N-(3',4'-dichloro-5-fluorobiphenyl-2-yl)-1-methyl-3-trifluoromethyl-1H-pyrazol-4-ylcarboxamide,
N-(3',4'-difluoro-5-fluorobiphenyl-2-yl)-1-methyl-3-trifluoromethyl-1H-pyrazol-4-ylcarboxamide,
N-(3',4'-dichloro-5-fluorobiphenyl-2-yl)-1-methyl-3-difluoromethyl-1H-pyrazol-4-ylcarboxamide,
N-(3',4'-difluoro-5-fluorobiphenyl-2-yl)-1-methyl-3-difluoromethyl-1H-pyrazol-4-ylcarboxamide,
N-(3',4'-dichloro-5-fluorobiphenyl-2-yl)-1,3-dimethyl-1H-pyrazol-4-ylcarboxamide,
N-(3'-chloro-4'-fluoro-5-fluorobiphenyl-2-yl)-1-methyl-3-difluoromethyl-1H-pyrazol-4-ylcarboxamide,
N-(4'-fluoro-4-fluorobiphenyl-2-yl)-1-methyl-3-trifluoromethyl-1H-pyrazol-4-ylcarboxamide,
N-(4'-fluoro-5-fluorobiphenyl-2-yl)-1-methyl-3-trifluoromethyl-1H-pyrazol-4-ylcarboxamide,
N-(4'-chloro-5-fluorobiphenyl-2-yl)-1-methyl-3-trifluoromethyl-1H-pyrazol-4-ylcarboxamide,
N-(4'-methyl-5-fluorobiphenyl-2-yl)-1-methyl-3-trifluoromethyl-1H-pyrazol-4-ylcarboxamide,
N-(4'-fluoro-5-fluorobiphenyl-2-yl)-1,3-dimethyl-1H-pyrazol-4-ylcarboxamide,
N-(4'-chloro-5-fluorobiphenyl-2-yl)-1,3-dimethyl-1H-pyrazol-4-ylcarboxamide,
N-(4'-methyl-5-fluorobiphenyl-2-yl)-1,3-dimethyl-1H-pyrazol-4-ylcarboxamide,
N-(4'-fluoro-6-fluorobiphenyl-2-yl)-1-methyl-3-trifluoromethyl-1H-pyrazol-4-ylcarboxamide,
N-[2-(1,1,2,3,3,3-hexafluoropropoxy)phenyl]-3-difluoromethyl-1-methyl-1H-pyrazol-4-ylcarboxamide,
N-[4'-(trifluoromethylthio)biphenyl-2-yl]-3-difluoromethyl-1-methyl-1H-pyrazol-4-ylcarboxamide,
N-[4'-(trifluoromethylthio)biphenyl-2-yl]-1-methyl-3-trifluoromethyl-1-methyl-1H-pyrazol-4-ylcarboxamide,
3-(difluoromethyl)-1-methyl-N-[1,2,3,4-tetrahydro-9-(1-methylethyl)-1,4-methanonaphthalen-5-yl]-1H-pyrazol-4-ylcarboxamide,
N-(3'-chloro-5-fluorobiphenyl-2-yl)-3-(difluoromethyl)-1-methylpyrazol-4-ylcarboxamide,
N-(4'-chloro-5-fluorobiphenyl-2-yl)-3-(difluoromethyl)-1-methylpyrazol-4-ylcarboxamide,
N-(4'-chlorobiphenyl-2-yl)-3-(difluoromethyl)-1-methylpyrazol-4-ylcarboxamide,
N-(4'-bromobiphenyl-2-yl)-3-(difluoromethyl)-1-methylpyrazol-4-ylcarboxamide,
N-(4'-iodobiphenyl-2-yl)-3-(difluoromethyl)-1-methylpyrazol-4-ylcarboxamide,
N-(3',5'-difluorobiphenyl-2-yl)-3-(difluoromethyl)-1-methylpyrazol-4-ylcarboxamide,
N-(2-chloro-4-fluorophenyl)-3-(difluoromethyl)-1-methylpyrazol-4-ylcarboxamide,
N-(2-bromo-4-fluorophenyl)-3-(difluoromethyl)-1-methylpyrazol-4-ylcarboxamide,
N-(2-iodo-4-fluorophenyl)-3-(difluoromethyl)-1-methylpyrazol-4-ylcarboxamide or
N-[2-(1,3-dimethylbutyl)phenyl]-1,3-dimethyl-5-fluoro-1H-pyrazol-4-ylcarboxamide.

7. The process according to claim 1, wherein the reaction of II with III is undertaken at a pressure of from 200 to 600 mbar.

8. The process according to claim 1, wherein the reaction of II with III is undertaken at from 20 to 120°C.

9. The process according to any of claims 1 to 8, wherein the acrylamine (III) is added in portions or continuously to the surface of the solution of acid chloride (II) or directly into the solution of acid chloride (II) as an "immersed mode of reaction".

## Revendications

1. Procédé pour la préparation d'arylcarboxamides de formule (I) 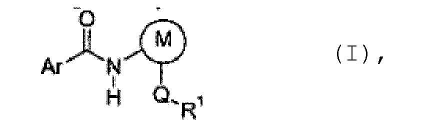 dans laquelle les substituants ont les significations suivantes :
Ar représente un cycle phényle, pyridyle ou pyrazolyle une à trois fois substitué, les substituants étant choisis chacun indépendamment parmi des atomes d'halogène, des groupes alkyle en C₁-C₄ et halogénoalkyle en C₁-C₄ ;
M représente un groupe thiényle ou phényle, qui peut porter un substituant halogène ;
Q représente une liaison directe, le groupe cyclopropène, un cycle bicyclo[2.2.1]heptane ou bicyclo[2.2.1]heptène soudé ;
R¹ représente un atome d'hydrogène ou d'halogène, un groupe alkyle en C₁-C₆, alcoxy en C₁-C₄, halogénoalcoxy en C₁-C₄, un groupe phényle une à trois fois substitué, les substituants étant choisis chacun indépendamment parmi des atomes d'halogène et le groupe trifluorométhylthio, ou cyclopropyle ;
par mise en réaction d'un chlorure d'acide de formule (II) 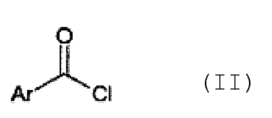 avec une arylamine (III) 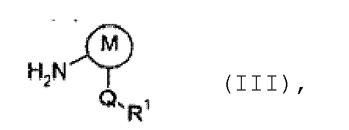 dans un solvant non aqueux convenable,
**caractérisé en ce qu'**en absence d'une base auxiliaire
a) on dispose au préalable le chlorure d'acide (II),
b) on règle une pression de 0 à 700 mbars,
c) on ajoute l'arylamine (III) par addition dosée, de manière que le rapport molaire de (II) à (III) vaille de 0,9 : 1 à 1,1 : 1 et
d) on isole le produit recherché.

2. Procédé selon la revendication 1, dans lequel Ar est un cycle phényle, pyridyle ou pyrazolyle 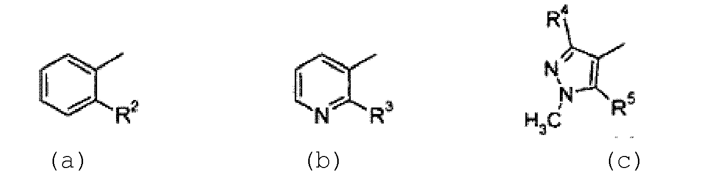 et
R² représente un atome d'halogène, le groupe méthyle ou trifluorométhyle ;
R³ représente un atome d'halogène ;
R⁴ représente un groupe alkyle en C₁-C₄ ou halogénoalkyle en C₁-C₄ et
R⁵ représente un atome d'hydrogène ou d'halogène.

3. Procédé selon la revendication 1, dans lequel M représente le groupe phényle, Q le groupe cyclo-propylène et R¹ le groupe cyclopropyle.

4. Procédé selon la revendication 1, dans lequel M représente le groupe phényle, Q représente une liaison et R¹ représente le groupe isopropoxy ou un groupe phényle 1 à 3 fois substitué, les substituants étant choisis chacun indépendamment parmi des atomes d'halogène et le groupe trifluorométhylthio.

5. Procédé selon la revendication 1, dans lequel M représente un groupe phényle substitué par halogène, Q représente une liaison et R¹ représente un atome d'hydrogène ou un groupe phényle 1 à 3 fois substitué par halogène.

6. Procédé selon la revendication 1, dans lequel l'arylcarboxamide est le bénodanil, le bixafène, le boscalide, le flutolanil, le mépronil, le penthiopyrade,
le N-(2-bicyclopropyl-2-yl-phényl)-3-difluorométhyl-1-méthylpyrazol-4-yl-carboxamide,
le N-(3',4',5'-trifluorobiphényl-2-yl)-1,3-diméthyl-pyrazol-4-yl-carboxamide,
le N-(3',4',5'-trifluorobiphényl-2-yl)-1,3-diméthyl-5-fluoro-pyrazol-4-yl-carboxamide,
le N-(3',4',5'-trifluorobiphényl-2-yl)-5-chloro-1,3-diméthyl-pyrazol-4-yl-carboxamide,
le N-(3',4',5'-trifluorobiphényl-2-yl)-3-fluorométhyl-1-méthyl-pyrazol-4-yl-carboxamide,
le N-(3',4',5'-trifluorobiphényl-2-yl)-3-(chlorofluorométhyl)-1-méthyl-pyrazol-4-yl-carboxamide,
le N-(3',4',5'-trifluorobiphényl-2-yl)-3-difluorométhyl-1-méthyl-pyrazol-4-yl-carboxamide,
le N-(3',4',5'-trifluorobiphényl-2-yl)-3-difluorométhyl-5-fluoro-1-méthyl-pyrazol-4-yl-carboxamide,
le N-(3',4',5'-trifluorobiphényl-2-yl)-5-chloro-3-difluorométhyl-1-méthyl-pyrazol-4-yl-carboxamide,
le N-(3',4',5'-trifluorobiphényl-2-yl)-3-(chlorodifluorométhyl)-1-méthyl-pyrazol-4-yl-carboxamide,
le N-(3',4',5'-trifluorobiphényl-2-yl)-1-méthyl-3-trifluorométhyl-pyrazol-4-yl-carboxamide,
le N-(3',4',5'-trifluorobiphényl-2-yl)-5-fluoro-1-méthyl-3-trifluorométhyl-pyrazol-4-yl-carboxamide,
le N-(3',4',5'-trifluorobiphényl-2-yl)-5-chloro-1-méthyl-3-trifluorométhyl-pyrazol-4-yl-carboxamide,
le N-(2',4',5'-trifluorobiphényl-2-yl)-1,3-diméthylpyrazol-4-yl-carboxamide,
le N-(2',4',5'-trifluorobiphényl-2-yl)-1,3-diméthyl-5-fluoropyrazol-4-yl-carboxamide,
le N-(2',4',5'-trifluorobiphényl-2-yl)-5-chloro-1,3-diméthyl-pyrazol-4-yl-carboxamide,
le N-(2',4',5'-trifluorobiphényl-2-yl)-3-fluorométhyl-1-méthyl-pyrazol-4-yl-carboxamide,
le N-(2',4',5'-trifluorobiphényl-2-yl)-3-(chlorofluorométhyl)-1-méthyl-pyrazol-4-yl-carboxamide,
le N-(2',4',5'-trifluorobiphényl-2-yl)-3-difluorométhyl-1-méthyl-pyrazol-4-yl-carboxamide,
le N-(2',4',5'-trifluorobiphényl-2-yl)-3-difluorométhyl-5-fluoro-1-méthylpyrazol-4-yl-carboxamide,
le N-(2',4',5'-trifluorobiphényl-2-yl)-5-chloro-3-difluorométhyl-1-méthyl-pyrazol-4-yl-carboxamide,
le N-(2',4',5'-trifluorobiphényl-2-yl)-3-(chlorodifluorométhyl)-1-méthyl-pyrazol-4-yl-carboxamide,
le N-(2',4',5'-trifluorobiphényl-2-yl)-1-méthyl-3-trifluorométhyl-pyrazol-4-yl-carboxamide,
le N-(2',4',5'-trifluorobiphényl-2-yl)-5-fluoro-1-méthyl-3-trifluorométhyl-pyrazol-4-yl-carboxamide,
le N-(2',4',5'-trifluorobiphényl-2-yl)-5-chloro-1-méthyl-3-trifluorométhyl-pyrazol-4-yl-carboxamide,
le N-(3',4'-dichloro-3-fluorobiphényl-2-yl)-1-méthyl-3-trifluorométhyl-1H-pyrazol-4-yl-carboxamide,
le N-(3',4'-dichloro-3-fluorobiphényl-2-yl)-1-méthyl-3-difluorométhyl-1H-pyrazol-4-yl-carboxamide,
le N-(3',4'-difluoro-3-fluorobiphényl-2-yl)-1-méthyl-3-trifluorométhyl-1H-pyrazol-4-carboxamide,
le N-(3',4'-difluoro-3-fluorobiphényl-2-yl)-1-méthyl-3-difluorométhyl-1H-pyrazol-4-yl-carboxamide,
le N-(3'-chloro-4'-fluoro-3-fluorobiphényl-2-yl)-1-méthyl-3-difluorométhyl-1H-pyrazol-4-yl-carboxamide,
le N-(3',4'-dichloro-4-fluorobiphényl-2-yl)-1-méthyl-3-trifluorométhyl-1H-pyrazol-4-yl-carboxamide,
le N-(3',4'-difluoro-4-fluorobiphényl-2-yl)-1-méthyl-3-trifluorométhyl-1H-pyrazol-4-yl-carboxamide,
le N-(3',4'-dichloro-4-fluorobiphényl-2-yl)-1-méthyl-3-difluorométhyl-1H-pyrazol-4-yl-carboxamide,
le N-(3',4'-difluoro-4-fluorobiphényl-2-yl)-1-méthyl-3-difluorométhyl-1H-pyrazol-4-yl-carboxamide,
le N-(3'-chloro-4'-fluoro-4-fluorobiphényl-2-yl)-1-méthyl-3-difluorométhyl-1H-pyrazol-4-yl-carboxamide,
le N-(3',4'-dichloro-5-fluorobiphényl-2-yl)-1-méthyl-3-trifluorométhyl-1H-pyrazol-4-yl-carboxamide,
le N-(3',4'-difluoro-5-fluorobiphényl-2-yl)-1-méthyl-3-trifluorométhyl-1H-pyrazol-4-yl-carboxamide,
le N-(3',4'-dichloro-5-fluorobiphényl-2-yl)-1-méthyl-3-difluorométhyl-1H-pyrazol-4-yl-carboxamide,
le N-(3',4'-difluoro-5-fluorobiphényl-2-yl)-1-méthyl-3-difluorométhyl-1H-pyrazol-4-yl-carboxamide,
le N-(3',4'-dichloro-5-fluorobiphényl-2-yl)-1,3-diméthyl-1H-pyrazol-4-yl-carboxamide,
le N-(3'-chloro-4'-fluoro-5-fluorobiphényl-2-yl)-1-méthyl-3-difluorométhyl-1H-pyrazol-4-yl-carboxamide,
le N-(4'-fluoro-4-fluorobiphényl-2-yl)-1-méthyl-3-trifluorométhyl-1H-pyrazol-4-yl-carboxamide,
le N-(4'-fluoro-5-fluorobiphényl-2-yl)-1-méthyl-3-trifluorométhyl-1H-pyrazol-4-yl-carboxamide,
le N-(4'-chloro-5-fluorobiphényl-2-yl)-1-méthyl-3-trifluorométhyl-1H-pyrazol-4-yl-carboxamide,
le N-(4'-méthyl-5-fluorobiphényl-2-yl)-1-méthyl-3-trifluorométhyl-1H-pyrazol-4-yl-carboxamide,
le N-(4'-fluoro-5-fluorobiphényl-2-yl)-1,3-diméthyl-1H-pyrazol-4-yl-carboxamide,
le N-(4'-chloro-5-fluorobiphényl-2-yl)-1,3-diméthyl-1H-pyrazol-4-yl-carboxamide,
le N-(4'-méthyl-5-fluorobiphényl-2-yl)-1,3-diméthyl-1H-pyrazol-4-yl-carboxamide,
le N-(4'-fluoro-6-fluorobiphényl-2-yl)-1-méthyl-3-trifluorométhyl-1H-pyrazol-4-yl-carboxamide,
le N-[2-(1,1,2,3,3,3-hexafluoropropoxy)-phényl]-3-difluorométhyl-1-méthyl-1H-pyrazol-4-yl-carboxamide,
le N-[4'-(trifluorométhylthio)-biphényl-2-yl]-3-difluorométhyl-1-méthyl-1H-pyrazol-4-yl-carboxamide,
le N-[4'-(trifluorométhylthio)-biphényl-2-yl]-1-méthyl-3-trifluorométhyl-1-méthyl-1H-pyrazol-4-yl-carboxamide,
le 3-(difluorométhyl)-1-méthyl-N-[1,2,3,4-tétrahydro-9-(1-méthyléthyl)-1,4-méthanonaphtalén-5-yl]-1H-pyrazol-4-yl-carboxamide,
le N-(3'-chloro-5-fluorobiphényl-2-yl)-3-(difluorométhyl)-1-méthylpyrazol-4-yl-carboxamide,
le N-(4'-chloro-5-fluorobiphényl-2-yl)-3-(difluorométhyl)-1-méthylpyrazol-4-yl-carboxamide,
le N-(4'-chlorobiphényl-2-yl)-3-(difluorométhyl)-1-méthylpyrazol-4-yl-carboxamide,
le N-(4'-bromobiphényl-2-yl)-3-(difluorométhyl)-1-méthylpyrazol-4-yl-carboxamide,
le N-(4'-iodobiphényl-2-yl)-3-(difluorométhyl)-1-méthylpyrazol-4-yl-carboxamide,
le N-(3',5'-difluorobiphényl-2-yl)-3-(difluorométhyl)-1-méthylpyrazol-4-yl-carboxamide,
le N-(2-chloro-4-fluoro-phényl)-3-(difluorométhyl)-1-méthylpyrazol-4-yl-carboxamide,
le N-(2-bromo-4-fluoro-phényl)-3-(difluorométhyl)-1-méthylpyrazol-4-yl-carboxamide,
le N-(2-iodo-4-fluoro-phényl)-3-(difluorométhyl)-1-méthylpyrazol-4-yl-carboxamide ou
le N-[2-(1,3-diméthylbutyl)-phényl]-1,3-diméthyl-5-fluoro-1H-pyrazol-4-yl-carboxamide.

7. Procédé selon la revendication 1, **caractérisé en ce qu'**on effectue la réaction de II avec III sous une pression de 200 à 600 mbars.

8. Procédé selon la revendication 1, **caractérisé en ce qu'**on effectue la réaction de II avec III à une température de 20 à 120 °C.

9. Procédé selon l'une quelconque des revendications 1 à 8, **caractérisé en ce que** l'addition d'arylamine (III) s'effectue par portions ou en continu sur la surface de la solution du chlorure d'acide (II) ou directement dans la solution du chlorure d'acide (II) en tant que « conduite de réaction en immersion ».
